# FASCICULE DE BREVET EUROPEEN

(11) **EP 3 400 058 B1**
(45) Date de publication et mention de la délivrance du brevet: **24.06.2020**
(21) Numéro de dépôt: 17700149.2
(22) Date de dépôt: 06.01.2017
(51) Int. Cl.: A61N 5/06, A61B 18/20, A61B 18/00

(54) **DISPOSITIF DE TRAITEMENT DERMATOLOGIQUE**
VORRICHTUNG FÜR DERMATOLOGISCHE BEHANDLUNGEN
DERMATOLOGICAL TREATMENT DEVICE

(30) Priorité: 07.01.2016 FR 1600037
(43) Date de publication de la demande: 14.11.2018
(73) Titulaire: Urgo Recherche Innovation et Développement, 21300 Chenove (FR)
(72) Inventeur: LAMOISE, Michel, 21110 Bessey Les Cîteaux (FR); LE LOUS, Guirec, 75007 Paris (FR)
(74) Mandataire: Barbot, Willy
(86) Numéro de dépôt international: PCT/EP2017/000014
(87) Numéro de publication internationale: WO 2017/118607

(56) Documents cités:
- WO-A1-2011/080574
- DE-A1-102013 017 912
- FR-A1- 2 938 179

## Description

### Domaine de l'invention

La présente invention concerne un dispositif de traitement dermatologique comprenant une tête laser apte à tirer un faisceau laser.

### Art antérieur

On connait des dispositifs de traitement dermatologiques, lesquels sont typiquement employés pour créer un échauffement déterminé et localisé d'une zone cible correspondant à une plaie d'un patient, laquelle comprend des tissus dermiques, et ceci afin d'en accélérer la cicatrisation. Un tel dispositif de traitement dermatologique est par exemple illustré par le dispositif décrit dans la demande internationale PCT WO 2009/071592 de la Demanderesse.

Pour qu'un tel effet de cicatrisation se produise de manière optimale, il convient que le tir laser échauffe les tissus dermiques éclairés par celui-ci jusqu'à ce qu'ils approchent une température optimale (comprise entre 45 et 55°C), mais sans dépasser une température maximale (de l'ordre de 60°C) pouvant occasionner des brûlures ou des dommages irréversibles des tissus dermiques.

En vue de déterminer la température des tissus dermiques durant le tir, il est possible d'adjoindre un pyromètre au dispositif de traitement dermatologique. Un tel dispositif est par exemple décrit dans la demande internationale PCT WO 2011/080574 de la Demanderesse.

Maintenant, et en vue de contrôler la température de la peau au niveau de la zone cible du tir laser, il convient d'employer un moyen de pilotage du tir laser apte à sélectivement activer ou désactiver un tir laser.

Il est connu dans le domaine des dispositifs de traitement dermatologique de contrôler la quantité de chaleur transmise aux tissus au moyen de tir laser en limitant la durée d'un tir à une valeur constante. Ainsi un précédent modèle de la demanderesse applique des tirs à puissance constante, par exemple de 6W, selon une durée configurable parmi deux valeurs : 10 ou 13s. Maintenant, une telle approche néglige trop de paramètres, tels que la variabilité des comportements de la peau d'un patient à un autre, et conduit à une trop grande variabilité de la température atteinte. Deux risques existent alors : une température trop importante de la peau est atteinte occasionnant des brûlures ou, à l'inverse, une température trop faible rend le traitement inefficace. Il convient donc de proposer un moyen de pilotage du tir laser plus précis.

### Sommaire de l'invention

L'invention est définie dans les revendications attenantes. La présente invention remédie à ces différents inconvénients et propose un moyen de pilotage apte à contrôler le tir laser en fonction de la température de la peau et d'une durée de tir variable.

L'invention a pour objet un dispositif de traitement dermatologique comprenant une tête laser apte à tirer un faisceau laser en direction d'une zone cible de la peau d'un patient, un pyromètre apte à mesurer la température (T) de la peau au niveau de ladite zone cible, un minuteur apte à mesurer la durée (D) du tir du laser, et un moyen de pilotage apte à sélectivement activer ou désactiver un tir laser, où le moyen de pilotage est configuré pour désactiver le tir laser lorsque la durée (D) du tir atteint un seuil de durée en secondes (Sd) tel que déterminé par la fonction affine de la forme Sd=(To-T-b)/C, avec T étant la température (T) mesurée de la peau, To une température objective, b étant un offset de température et C un coefficient d'échauffement moyen de la peau.

La température objective To correspond à l'objectif de température ou température visée.

L'offset de température correspond à une valeur corrective, laquelle valeur a été déterminée par les inventeurs de sorte de tenir compte de la sensibilité/précision des matériels notamment. Il peut s'agir d'une constante ou d'une valeur variant en fonction de la température, de préférence cet offset de température est une constante.

Le coefficient d'échauffement moyen de la peau est une valeur rendant compte de la variation de la température T mesurée de la peau en fonction du temps (en °C par s⁻¹), cette valeur peut être déterminée pour une population de patients donnée.

Dans le dispositif selon l'invention, la variation de la température de la peau est suivie avec le temps et de façon dynamique par le pyromètre. Avantageusement, le seuil de durée Sd est déterminé immédiatement avant ou immédiatement après le début du tir laser, de préférence immédiatement après le début du tir laser, c'est-à-dire de préférence dans la seconde suivant le début du tir laser. Avantageusement encore, le seuil de durée peut être réévalué lors du tir laser à une, à deux ou plusieurs reprises.

Avantageusement, le moyen de pilotage est également configuré pour désactiver le tir laser lorsque la température (T) atteint un seuil de température (St).

Le moyen de pilotage est alors configuré pour désactiver le laser dès lors que l'un des deux seuils est atteint, soit que la température (T) de la peau a atteint le seuil de température (St) soit que la durée du tir a atteint la durée maximale Sd tel que déterminée par la fonction affine définit précédemment.

L'invention a également pour objet un système de traitement dermatologique, ledit système comprenant un dispositif tel que décrit ci-dessus et des moyens d'interaction entre ladite tête laser et la zone de peau à traiter, lesdits moyens d'interaction étant agencés pour coopérer avec lesdits moyens d'asservissement.

La divulgation montre également un procédé de traitement dermatologique mettant en œuvre un dispositif ou un système tel que décrit précédemment.

### Descriptif des figures

La figure 1 schématise le dispositif selon l'invention.
La figure 2 représente un diagramme de la durée en fonction de la température.

### Descriptif détaillé de l'invention

D'autres caractéristiques, détails et avantages de l'invention ressortiront plus clairement de la description détaillée donnée ci-après à titre indicatif.

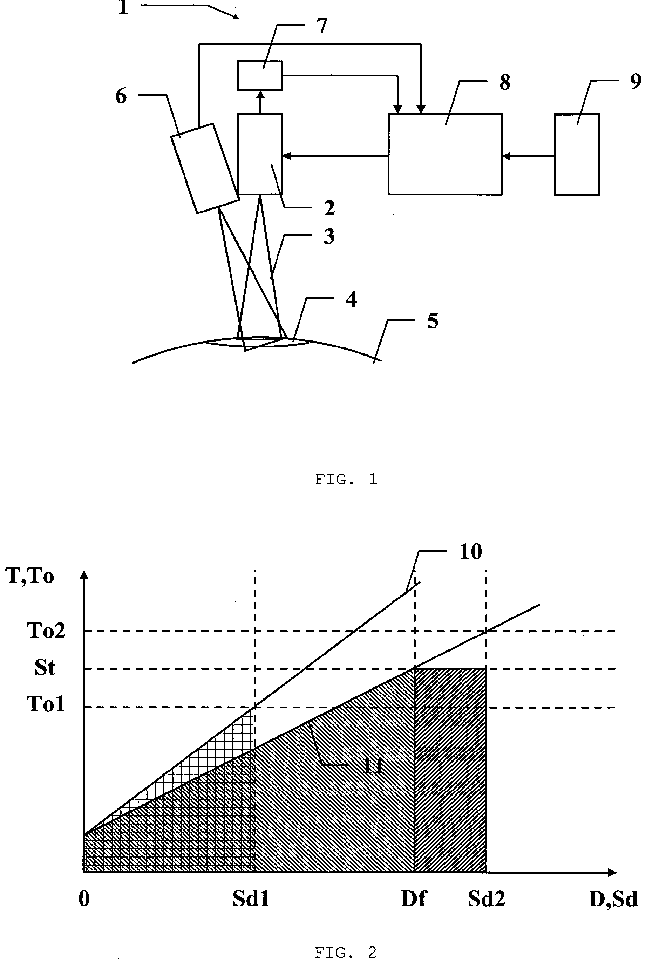

Tel qu'illustré à la figure 1, le dispositif 1 de traitement dermatologique comprend une tête laser 2, un pyromètre 6, un minuteur 7 et un moyen de pilotage 8. La tête laser 2 est apte à tirer un faisceau laser 3 en direction d'une zone cible 4 située sur la peau 5 d'un patient. Cette illumination a pour but de produire un échauffement contrôlé de la peau 5 au niveau de la zone cible 4. Le pyromètre 6 est apte à mesurer la température T de la peau 5 au niveau de ladite cible 4, soit au droit de la surface de peau recevant le tir du faisceau laser 3. Le minuteur 7 est apte à déterminer la durée D de tir de la tête laser 2. Le moyen de pilotage 8 est apte à contrôler la configuration et le fonctionnement de la tête laser 2. Il est ainsi en charge de l'activation ou de la désactivation du tir laser et tout particulièrement de la gestion de la sécurité associée.

Le moyen de pilotage 8 peut être électronique, informatique ou une combinaison des deux. La configuration du moyen de pilotage 8 est assurée, par câblage, ou plus typiquement, par un programme ou logiciel que le moyen de pilotage 8 est apte à exécuter.

Le dispositif 1 peut encore comprendre une interface homme machine 9. Cette interface homme machine peut permettre à un opérateur de configurer le dispositif 1 en indiquant les réglages souhaités et permet de commander son utilisation. Lors de l'utilisation du dispositif 1, le début ou activation d'un tir laser est typiquement déclenché par une commande de l'opérateur. Par contre, afin de sécuriser, la fin ou désactivation du tir est commandée par le moyen de pilotage 8. Ainsi la quantité d'énergie maximale transmise à la zone cible 4 reste en permanence sous le contrôle du moyen de pilotage 8.

Avantageusement, le faisceau laser présente une longueur d'onde comprise entre 0,8pm et 2µm, de préférence entre 0,9 et 1,8 µm et de manière particulièrement préférée entre 1 et 1,6 µm.

Maintenant, et dans un mode de réalisation préférentiel, le faisceau laser présente une longueur d'onde de l'ordre de 1 200 nm (p. ex. 1210 nm).

Selon une caractéristique avantageuse, le moyen de pilotage 8 est configuré pour désactiver le tir laser dès la survenue de l'une au moins de deux conditions. Une première condition est liée à la température T, de la zone cible 4 telle que mesurée par le pyromètre 6. La première condition d'arrêt du tir laser est réalisée lorsque la température T atteint un seuil de température St.

Une deuxième condition est liée à la durée D du tir laser telle que mesurée par le minuteur 7. La deuxième condition d'arrêt du tir laser est réalisée lorsque la durée du tir laser atteint un seuil de durée Sd.

Le tir laser est arrêté dès que l'une au moins de ces deux conditions, et donc la plus rapide, se réalise.

Le seuil de température St est avantageusement une constante.

Selon un mode de réalisation spécifique, le seuil de température St appliqué par le moyen de pilotage 8 pondéré de l'offset de température (St - b) est inférieur ou égal à la température objective To et le coefficient d'échauffement moyen C est inférieur à 3, de préférence inférieur à 2,2.

Avantageusement, le seuil de température St est alors compris entre 50 et 56°C, le coefficient d'échauffement moyen C est compris entre 1,10 et 2,10 de préférence compris entre 1,30 et 1, 90, l'offset b étant compris entre 2,5 et 4,5, de préférence égal à 3,5 et la température objective est comprise entre 53,5 et 59°C, de préférence égale à 56°C.

Avantageusement, le seuil de température St est égal à 50°C, le coefficient d'échauffement moyen C est égal à 1,6, l'offset b est égal à 3,5 et la température objective est égale à 56°C.

Ici la température T est régulièrement mesurée par le pyromètre 6 et actualisée durant le déroulement d'un tir laser. Cette valeur de température T actualisée est, à chaque réactualisation, comparée avec la valeur seuil de température St afin de tester la réalisation de la première condition.

Le seuil de durée Sd est avantageusement une fonction décroissante de la température T. Ainsi, plus la température T est initialement élevée et plus la durée Sd du tir laser est courte.

Pour ce qui est de la mise en œuvre des deux sécurités (en lien avec St et Sd respectivement) induisant la désactivation du laser, on a deux scénarios possibles.

Dans le scénario où c'est la sécurité relative à la température seuil St qui induit la désactivation du laser, la température T est mesurée une fois au début du tir laser et est utilisée, via ladite fonction, pour déterminer une durée maximale Sd du tir. La température de la peau est régulièrement mesurée à partir de l'instant de début du tir laser. Dès lors que la peau atteint la température seuil (St) et même si la durée du tir n'a pas atteint le seuil Sd, le tir du laser est stoppé (première sécurité).

Dans le second scénario, où c'est la sécurité relative à la durée maximale du tir qui induit la désactivation du laser, la température T est mesurée également une fois au début du tir laser et est utilisée, là encore via ladite fonction, pour déterminer une durée maximale Sd du tir. La température de la peau est régulièrement mesurée à partir de l'instant de début du tir laser. Dès lors que la durée maximale de tir Sd est atteinte, et même si la température de la peau n'a pas atteint la température seuil, le tir du laser est là encore stoppé (seconde sécurité). Maintenant, et pour ce qui est de la durée du tir, il est envisageable de la réévaluer une ou plusieurs fois pendant le tir. Avec cette réévaluation, il est possible de réaliser un suivi au plus près de toute variation pouvant se produire dans le déroulement du tir laser et/ou dans le comportement de la peau en réponse.

La fonction qui détermine le seuil de durée est avantageusement une fonction affine de la forme Sd=(To-T-b)/C, avec Sd le seuil de durée, T la température, To une température objective, b étant un offset de température et C un coefficient d'échauffement moyen de la peau.

Avantageusement le coefficient d'échauffement moyen C et la température objective To sont des constantes.

Ainsi calculé, en considérant que l'échauffement de la peau 5 peut être modélisé par un modèle linéaire de gain égal au coefficient d'échauffement moyen C, le seuil de durée Sd constitue une estimation du temps nécessaire à la peau 5 pour passer de la température T à la température objective To. Si tout se déroule comme prévu, la deuxième condition d'arrêt est réalisée lorsque la peau 5 atteint la température objective (moins l'offset de température).

La figure 2 présente un diagramme température T / durée D. Dans un tel diagramme peut être figuré un point de fonctionnement de la peau 5 soumise à un tir laser et son évolution dans la durée. Une limite de température St détermine la première condition d'arrêt d'un tir laser. Un point de fonctionnement ne peut se situer au-dessus de cette limite St horizontale. Une limite de durée Sd détermine la deuxième condition d'arrêt d'un tir laser. Un point de fonctionnement ne peut se situer à droite de cette limite verticale.

Il apparait que les deux conditions de terminaison du tir laser sont intimement liées en ce qu'elles contribuent, ensemble, à sécuriser le dispositif 1. Ainsi tous les paramètres, de la première condition : St, et de la deuxième condition : C, To, doivent être considérés et déterminés ensemble, afin de coopérer efficacement.

Selon un premier mode de réalisation, la seconde condition, déterminant un seuil de durée Sd de la forme Sd= (To-T-b)/C, emploie un coefficient C plus élevé et une température objective To inférieure au seuil de température St. La température T de la peau suit alors un échauffement C plus rapide selon la courbe 10, de pente C plus importante. La température objective est To1, inférieure au seuil de température St. Il s'ensuit que le seuil de durée est déterminé égal à Sd1.

Dans un tel mode de réalisation, la température objective To1 est juste atteinte à la durée Sd1, où le tir laser est arrêté. Ici c'est la deuxième condition, de durée, qui limite le tir laser. La première condition, de température, n'est ici présente qu'en secours, afin par exemple d'éviter une surchauffe de la peau pouvant entraîner une brûlure. Les points de fonctionnement peuvent être situés dans la zone quadrillée.

Un exemple fonctionnel et utilisable d'un tel mode de réalisation, utilise les paramètres suivants : un seuil de température St égal à 53°C, un coefficient d'échauffement moyen C égal à 1,60, un offset b égal à 3,5 et une température objective To égale à 56°C. Cet exemple est fonctionnel et utilisé expérimentalement. Cependant quelques incidents rencontrés ont conduit à le modifier.

Selon un deuxième mode de réalisation, la seconde condition, déterminant un seuil de durée Sd de la forme Sd= (To-T-b)/C, emploie un coefficient C plus faible mais une température objective To supérieure au seuil de température St. La température T de la peau suit alors un échauffement C plus lent selon la courbe 11, de pente C plus faible. La température objective est To2, supérieure au seuil de température St. Il s'ensuit que le seuil de durée est déterminé égal à Sd2.

Dans un tel mode de réalisation, la température objective To2 n'est, en principe, pas atteinte. La montée en température est supposée plus lente et s'accompagne d'un seuil de durée, ici Sd2, plus long. Un tir dure potentiellement plus longtemps. Ici la première condition, de température, qui limite le tir laser, et le termine à la durée finale Df, correspondant à l'atteinte de la température St. La deuxième condition, de durée, est cependant aussi présente et permet, le cas échéant, de prolonger la durée du tir jusqu'à la durée Sd2, pour augmenter les chances d'atteindre la température St. Les points de fonctionnement peuvent être situés dans la zone hachurée selon un premier mode de hachures allant jusqu'à Df, qui peut ainsi s'étendre, au maximum, jusqu'à Sd2 et inclure la zone hachurée selon un deuxième mode de hachures.

Ce deuxième mode de réalisation augmente ainsi avantageusement, de manière significative, la probabilité d'atteindre le seuil de température St, qui est alors avantageusement fixé à une valeur optimale de traitement. Ceci permet d'obtenir une meilleure efficacité en ce qu'est ainsi prise en compte la variabilité du comportement thermique de la peau d'un patient à l'autre. Ce deuxième mode de réalisation offre une durée augmentée pour tenter d'atteindre une température efficace St. Ainsi, si un patient présente un coefficient d'échauffement inférieur au coefficient d'échauffement moyen C, sa réponse thermique plus lente est compensée par une durée de tir augmentée. Ceci augmente les chances de correctement traiter un tel patient.

Un exemple fonctionnel et utilisable d'un tel mode de réalisation, utilise les paramètres suivants : un seuil de température St égal à 50°C, un coefficient d'échauffement moyen C égal à 1,6, un offset b égal à 3,5 et une température objective To égale à 56°C. Le coefficient d'échauffement C est un coefficient moyen obtenu par une campagne de mesure réalisée sur une population de patients. Le précédent coefficient d'échauffement moyen C de 1,98 était déterminé au moyen d'une population majoritairement composée de patients sains. Le nouveau coefficient d'échauffement moyen C de 1,6 est plus réaliste en ce qu'il est déterminé au moyen d'une population majoritairement composée de patients susceptibles d'être traités par le dispositif 1.

L'utilisation du seuil de température St, non plus comme une sécurité mais comme une condition déterminant nominalement l'arrêt du tir laser conduit à revoir sa valeur à la baisse. A contrario, la température objective a été augmentée, afin d'augmenter le seuil de durée Sd et ainsi les chances d'atteindre le seuil de température St.

Les deux conditions d'arrêt de tir laser du dispositif 1 ne sont avantageusement pas configurables par l'opérateur, afin d'éviter tout risque de brûlure et/ou d'inefficacité du traitement.

Cependant une possibilité de configuration de la température objective To est avantageusement implantée dans le moyen de pilotage 8, pour permettre un réglage de type constructeur ou maintenance, accessible uniquement à un personnel habilité et connaissant les risques. Cependant la latitude de configuration de la température objective To est strictement limitée à un intervalle limité, selon une variation de +/- 0,7°C autour de la valeur nominale de To.

De manière préférentielle une autre sécurité peut encore être implantée dans le dispositif 1 de traitement dermatologique. Cette sécurité observe la vitesse de variation de la température T de la peau, telle que mesurée par le pyromètre 6 et commande un arrêt immédiat du tir laser si cette élévation de la température T de la peau est soit trop rapide soit trop lente relativement à sa valeur théorique.

Ceci permet de détecter un comportement atypique de la peau et ainsi éviter soit un manque d'efficacité du traitement en cas d'élévation de la température trop lente, soit à contrario un risque de brûlure en cas d'élévation de la température trop rapide.

La vitesse de variation de la température T de la peau est déterminée en observant la variation de la température T mesurée de la peau en fonction du temps / de la durée D. Cette variation est régulièrement mesurée durant une utilisation du dispositif 1 et comparée à sa valeur théorique. Cette valeur théorique, en reprenant un modèle linéaire D = ΔT/C, soit ΔT = C.D, tel que précédemment, est la pente de la courbe de variation de la température T en fonction de la durée D est égale au coefficient d'échauffement moyen C.

Aussi, dès que la variation de la température en fonction du temps est soit trop faible, soit trop importante, relativement à la valeur du coefficient d'échauffement moyen C retenue, le tir laser est immédiatement stoppé.

A titre indicatif, il est considéré qu'une valeur est trop importante ou trop faible si elle diffère de plus de 10% de sa valeur théorique.

L'invention a également pour objet un système de traitement dermatologique par faisceau laser, ledit système comprenant un dispositif tel que décrit précédemment et des moyens d'interaction entre ledit dispositif et la zone cible à traiter, lesdits moyens d'interaction étant agencés pour coopérer avec ledit moyen de pilotage.

Plus particulièrement, lesdits moyens d'interaction peuvent comprendre un support adhésif muni de moyens d'identification (p.ex. puce RFID) et susceptible d'être fixé à proximité de la zone cible à traiter, et communiquant avec une interface (p.ex. par radiofréquences) en liaison avec ledit moyen de pilotage.

De tels moyens d'interaction sont connus par les demandes internationales PCT WO 2007/080239 et PCT WO 2008/107563 et ne seront donc pas décrits ici plus en détail.

La divulgation permet de mettre en œuvre un procédé de traitement dermatologique comprenant les étapes consistant à:
- diriger le faisceau laser d'un dispositif tel que décrit précédemment sur une surface de la zone cible de peau à traiter d'un patient,
- mesurer, à l'aide du pyromètre décrit précédemment, la température de la surface de peau contenue dans son champ de vision, laquelle surface de peau est intégralement comprise dans la zone de peau traitée par ledit dispositif, et
- asservir ladite source lumineuse auxdits moyens de mesure de sorte que la température de la zone de peau traitée soit comprise entre 45 et 60°C.

## Revendications

1. Un dispositif (1) de traitement dermatologique comprenant une tête laser (2) apte à tirer un faisceau laser (3) en direction d'une zone cible (4) de la peau (5) d'un patient, un pyromètre (6) apte à mesurer la température (T) de la peau (5) au niveau de ladite zone cible (4), un minuteur (7) apte à mesurer la durée (D) du tir du laser, et un moyen de pilotage (8) apte à sélectivement activer ou désactiver un tir laser, dans lequel le pyromètre (6) permet de suivre la variation de la température de la peau (5) avec le temps et de façon dynamique et le moyen de pilotage (8) est configuré pour désactiver le tir laser lorsque la durée (D) du tir atteint un seuil de durée en secondes (Sd) **caractérisé en ce que** le seuil de durée (Sd) est déterminé immédiatement avant ou immédiatement après le début du tir laser par une fonction affine de la forme Sd=(To-T-b)/C, avec T étant la température (T) mesurée de la peau, To la température visée, b un offset de température correspondant à une valeur corrective et C un coefficient d'échauffement moyen de la peau qui rend compte de la variation de la température T mesurée de la peau en fonction du temps (en °C par s⁻¹).

2. Le dispositif selon la revendication **1,** où le moyen de pilotage (8) est également configuré pour désactiver le tir laser lorsque la température (T) atteint un seuil de température (St).

3. Le dispositif selon la revendication **2,** où le seuil de température (St) pondéré de l'offset de température (St - b) est inférieur ou égal à la température objective (To) et le coefficient d'échauffement moyen (C) est inférieur à 3, de préférence inférieur à 2,2.

4. Le dispositif selon la revendication **3,** où le seuil de température (St) est compris entre 50 et 56°C, l'offset de température b est compris entre 2,5 et 4,5 le coefficient d'échauffement moyen (C) est compris entre 1,1 et 2,1, et la température objective (To) est comprise entre 53,5 et 59°C.

5. Le dispositif selon la revendication **4,** où le coefficient d'échauffement moyen (C) est compris entre 1,3 et 1,9.

6. Le dispositif selon la revendication **5,** où le seuil de température (St) est égal à 50°C, le coefficient d'échauffement moyen (C) est égal à 1,6, l'offset de température b est égal à 3,5 et la température objective (To) est égale à 56°C.

7. Le dispositif selon l'une quelconque des revendications **1** à **6,** où la température objective (To) est configurable selon une variation de +/- 0,7°C autour de sa valeur nominale.

8. Le dispositif selon l'une quelconque des revendications **1** à **7,** où le moyen de pilotage est également configuré pour désactiver le tir laser lorsque la variation de la température (T) mesurée de la peau en fonction de la durée (D) est trop importante ou trop faible relativement à sa valeur théorique égale au coefficient d'échauffement moyen (C).

9. Le dispositif selon la revendication **8,** où trop importante ou trop faible signifie qu'elle diffère de plus de 10%.

10. Un système de traitement dermatologique par faisceau laser, ledit système comprenant :
i) le dispositif tel que défini à l'une quelconque des revendications **1** à **9,** et
ii) des moyens d'interaction entre ledit dispositif et la zone cible à traiter, lesdits moyens d'interaction étant agencés pour coopérer avec le moyen de pilotage.

11. Le dispositif selon la revendication 1, **caractérisé en ce que** le seuil de durée Sd est calculé immédiatement après le début du tir laser, c'est-à-dire de préférence dans la seconde suivant le début du tir laser.

12. Le dispositif selon la revendication 1, **caractérisé en ce que** le seuil de durée peut être réévalué lors du tir laser à une, à deux ou plusieurs reprises.

## Patentansprüche

1. Dermatologische Behandlungsvorrichtung (1), einen Laserkopf (2) umfassend, der in der Lage ist, einen Laserstrahl (3) in Richtung eines Zielbereiches (4) der Haut (5) eines Patienten abzugeben, ein Pyrometer (6), in der Lage, die Temperatur (T) der Haut (5) in Höhe des genannten Zielbereiches (4) zu messen, eine Schaltuhr (7), in der Lage, die Dauer (D) der Laserstrahlabgabe zu messen, und ein Leitmittel (8), geeignet, eine Laserstrahlabgabe wahlweise ein- oder auszuschalten, **dadurch gekennzeichnet, dass** das Pyrometer (6) erlaubt, die Änderungen der Temperatur der Haut (5) mit der Zeit und dynamisch zu verfolgen und dadurch, dass das genannte Leitmittel (8) dafür eingerichtet ist, die Laserstrahlabgabe auszuschalten, wenn die Dauer (D) der Abgabe einen Schwellenwert der Dauer (Sd) in Sekunden erreicht, der unmittelbar vor oder unmittelbar nach dem Beginn der Laserstrahlabgabe nach einer affinen Funktion der Form Sd = (To - T - b)/C bestimmt wurde, in der T die gemessene Hauttemperatur (T) ist, To die angestrebte Temperatur, b ein Temperaturversatz, der einem Korrekturwert entspricht, und C ein mittlerer Erwärmungskoeffizient der Haut, der die Änderung der gemessenen Hauttemperatur T in Abhängigkeit von der Zeit (in °C*s⁻¹) berücksichtigt.

2. Vorrichtung nach Patentanspruch 1, in der das Leitmittel (8) auch dafür eingerichtet ist, die Laserstrahlabgabe auszuschalten, wenn die Temperatur (T) einen Schwellenwert der Temperatur (St) erreicht.

3. Vorrichtung nach Patentanspruch 2, in der der mit dem Temperaturversatz gewichtete Schwellenwert der Temperatur (St), also (St - b), kleiner oder gleich der objektiven Temperatur (To) ist und der mittlere Erwärmungskoeffizient (C) kleiner ist als 3, vorzugsweise kleiner als 2,2.

4. Vorrichtung nach Patentanspruch 3, in der der Schwellenwert der Temperatur (St) zwischen 50 und 56°C liegt, der Temperaturversatz b zwischen 2,5 und 4,5 liegt, der mittlere Erwärmungskoeffizient (C) zwischen 1,1 und 2,1 liegt und die objektive Temperatur (To) zwischen 53,5 und 59°C liegt.

5. Vorrichtung nach Patentanspruch 4, in der der mittlere Erwärmungskoeffizient (C) zwischen 1,3 und 1,9 liegt.

6. Vorrichtung nach Patentanspruch 5, in der der Schwellenwert der Temperatur (St) 50°C beträgt, der mittlere Erwärmungskoeffizient (C) 1,6 beträgt, der Temperaturversatz b 3,5 beträgt, und die objektive Temperatur (To) 56°C beträgt.

7. Vorrichtung nach irgendeinem der Patentansprüche 1 bis 6, in der die objektive Temperatur (To) entsprechend einer Schwankung um +/- 0,7°C um ihren Nennwert konfiguriert werden kann.

8. Vorrichtung nach irgendeinem der Patentansprüche 1 bis 7, ebenfalls dafür konfiguriert, die Laserstrahlabgabe auszuschalten, wenn die Änderung der gemessenen Hauttemperatur (T) in Abhängigkeit von der Dauer (D) zu groß ist oder zu klein gegenüber ihrem theoretischen Wert, der dem mittleren Erwärmungskoeffizienten (C) gleich ist.

9. Vorrichtung nach Patentanspruch 8, in der zu groß oder zu klein bedeutet, dass sie um mehr als 10% abweicht.

10. Dermatologisches System zur Laserstrahlbehandlung, umfassend:
i) eine Vorrichtung nach irgendeinem der Patentansprüche 1 bis 9, und
ii) Mittel zur Zusammenwirkung der genannten Vorrichtung mit dem zu behandelnden Zielbereich, wobei die genannten Mittel zur Zusammenwirkung dafür eingerichtet sind, mit dem Leitmittel zusammenzuwirken.

11. Vorrichtung nach Patentanspruch 1, **dadurch gekennzeichnet, dass** der Schwellenwert der Dauer (Sd) unmittelbar nach dem Beginn der Laserstrahlabgabe berechnet wird, d.h. vorzugsweise in der Sekunde, die auf den Beginn der Laserstrahlabgabe folgt.

12. Vorrichtung nach Patentanspruch 1, **dadurch gekennzeichnet, dass** der Schwellenwert der Dauer während der Laserstrahlabgabe einmal, zweimal oder mehrmals erneut bestimmt werden kann.

## Claims

1. A dermatological treatment device (1) comprising a laser head (2) capable of shooting a laser beam (3) towards a target region (4) of the skin (5) of a patient, a pyrometer (6) capable of measuring the temperature (T) of the skin (5) in said target region (4), a timer (7) capable of measuring the duration (D) of the laser shot, and a control means (8) capable of selectively activating or deactivating a laser shot, ***characterized in that*** the pyrometer (6) is able to measure the temperature of the skin (5) over time and in a dynamic way and **in that** the control means (8) is configured to deactivate the laser shot when the duration (D) of the shot reaches a duration threshold in seconds (Sd) as determined immediately before or immediately after the beginning of the laser shot by a linear function in the form Sd=(To-T-b)/C, where T is the measured temperature (T) of the skin, To is a target temperature, b is a temperature offset corresponding to a corrective value and C is an average heating coefficient of the skin which reflects the variation of the skin temperature T measured over time (in °C.s⁻¹)

2. The device according to claim 1, where the control means (8) is also configured to deactivate the laser shot when the temperature (T) reaches a temperature threshold (St).

3. The device according to claim 2, where the temperature threshold (St) weighted by the temperature offset (St - b) is less than or equal to the objective temperature (To) and the average heating coefficient (C) is less than 3, preferably less than 2.2.

4. The device according to claim 3, where the temperature threshold (St) is comprised between 50 and 56°C, the temperature offset b is comprised between 2.5 and 4.5, the average heating coefficient (C) is comprised between 1.1 and 2.1, and the objective temperature (To) is comprised between 53.5 and 59°C.

5. The device according to claim 4, where the average heating coefficient (C) is comprised between 1.3 and 1.9.

6. The device according to claim 5, where the temperature threshold (St) is equal to 50°C, the average heating coefficient (C) is equal to 1.6, the temperature offset b is equal to 3.5 and the objective temperature (To) is equal to 56°C.

7. The device according to any one of claims 1 to 6, where the objective temperature (To) is configurable according to a variation of +/- 0.7°C around its nominal value.

8. The device according to any one of claims 1 to 7, is also configured to deactivate the laser shot when the variation of the measured temperature (T) of the skin over the duration (D) is too high or too low relative to its theoretical value equal to the average heating coefficient (C) .

9. The device according to claim 8, where too high or too low means that it differs by more than 10%.

10. A dermatological treatment system by laser beam, said system comprising:
i) a device as defined in any one of claims 1 to 9, and
ii) interaction means between said laser head and the target zone to be treated, said interaction means being arranged to cooperate with said control means

11. The device according to claim 1, **characterized in that** the duration threshold (Sd) is calculated immediately after the beginning of the laser shot, that is preferably in the second following the beginning of the laser shot.

12. The device according to claim 1, **characterized in that** the duration threshold can be revaluated during the laser shot once, twice, or repeatedly.
